# EUROPEAN PATENT APPLICATION

(11) **EP 4 011 277 A1**
(43) Date of publication of application: **15.06.2022**
(21) Application number: 20213274.2
(22) Date of filing: 11.12.2020
(51) Int. Cl.: A61B 5/00, A61B 5/11, A61B 5/107

(54) **SYSTEM AND METHOD FOR DETERMINING A SLEEP POSTURE OF A SUBJECT**

(71) Applicant: Koninklijke Philips N.V., 5656 AG Eindhoven (NL)
(72) Inventor: SCHIPPER, Alphonsus Tarcisius Jozef Maria, 5656 AE Eindhoven (NL); DOS SANTOS DA FONSECA, Pedro Miguel Ferreira, 5656 AE Eindhoven (NL); BROUWER, Jan, 5656 AE Eindhoven (NL)
(74) Representative: Philips Intellectual Property & Standards

(57) **Abstract**

A system and method for determining a sleep posture of a subject uses a three-axis accelerometer coupled to the subject. A plurality of three-axis acceleration signals of the three-axis accelerometer are analyzed over time and from this analysis a reference craniocaudal rotation axis is derived. For an acceleration measurement of interest (i.e. at a particular point in time) a rotation angle can then be determined about the reference craniocaudal rotation axis, thus indicating a sleep posture relative to that reference craniocaudal rotation axis.

## Description

### FIELD OF THE INVENTION

This invention relates to systems and methods for determining a sleep posture of a subject. One application of interest is for treating sleep apnea, in particular when used as part of a positional sleep therapy system and method.

### BACKGROUND OF THE INVENTION

Sleep apnea is a multifactorial disorder. Age, gender, body weight, cardiac insufficiencies, pulmonary diseases, seasonal infections, life style contribute and worsen the disorder.

Obstructive sleep apnea (OSA) is characterized by a narrowing and collapse of the upper airway. Anatomical properties and features contribute mainly to the collapse. Beside static anatomical features, also dynamic alterations of the upper airway impede the narrowing of the airway. In particular, the gravitational displacement and swelling of soft tissue in the airway (palate, tongue, and epiglottis) narrow the airway and increase the Apnea Hypopnea index (AHI).

Central sleep apnea (CSA) is characterized by a decrease of the respiration drive. The decrease of neural activity leads to a decline in tidal volume, a fully cessation of airflow or a periodic breathing (Cheyne-Stokes Breathing).

One treatment approach for sleep apnea is to control the posture of the patient during sleep, in particular to ensure they sleep on their side rather than on their back.

A traditional method of positional sleep training is known as the "Tennis Ball Technique". With this technique, a tennis ball is sewn into the back of a shirt worn to bed by the subject so that the subject finds sleeping on his or her back uncomfortable.

Electronic positional sleep therapy devices are also well known for treating positional sleep apnea. Typical positional sleep therapy device use an accelerometer system to detect whether a subject is sleeping on his or her back and provide vibratory feedback to the subject that prompts the subject to change his or her posture.

In home positional therapy systems, the patients themselves place the accelerometer on the body, potentially causing variations in body position and in sensor orientation. In addition, the shape of the torso of the patients may vary significantly over the population. The combination of these two factors may cause large variations in the relation between the accelerometer orientation and the body posture, which may cause the pre-trained model to predict incorrect postures.

It would be desirable to calibrate the position of the sensor in a simple way so that accurate posture information is recorded without complicating the use of the system for the user.

### SUMMARY OF THE INVENTION

The invention is defined by the claims.

According to examples in accordance with an aspect of the invention, there is provided a system for determining a sleep posture of a subject, comprising:
a three-axis accelerometer for coupling to the subject; and
a controller,
wherein the controller is adapted to:
   determine, from a plurality of three-axis acceleration signals of the three-axis accelerometer over time, a reference craniocaudal rotation axis; and
   determine for an acceleration measurement of interest a rotation angle about the reference craniocaudal rotation axis

This system determines a rotation angle about a craniocaudal (head-to-toe) rotation axis by analyzing acceleration signals over time. In this way, the requirements on positioning of the sensor with respect to the body of the subject are relaxed, because the sensor orientation can be derived from the analysis of signals collected over time, such as during an initial period of monitoring in bed.

The reference craniocaudal rotation axis is for example assumed to lie in a horizontal plane. This enables the orientation of a reference craniocaudal rotation axis to be defined in 3D space.

It is thereby possible to make no assumptions on the position of the sensor on the body or on the orientation of the sensor. Instead, the reference axis of rotation is obtained by observing various postures over time, and an angle of rotation can be then calculated for each of the postures. Identifying the rotation axis and angles thereby allows for full freedom in the location and way of placement of the sensor.

The posture of the body during sleep is a parameter of interest in polysomnography. One application is for the diagnosis of positional sleep apnea as discussed above, in which apnea events occur more frequently in the supine posture than in other postures.

If a reference posture is required, such as is required for positional therapy for treating positional sleep apnea (where the supine posture must be known), then a reference posture may additionally be identified as explained below.

The controller may be adapted to determine the reference craniocaudal rotation axis by:
deriving a respective cross product vector for a plurality of pairs of the three-axis acceleration measurements; and
inverting selected cross product vectors such that all resulting cross product vectors are directed within a common semi-sphere; and
obtaining the reference craniocaudal rotation axis from the resulting cross product vectors.

The cross product of two vectors has a direction corresponding to a rotation axis between them, and in this case the rotation axis is the craniocaudal rotation axis (since this is the axis about which the body posture changes during sleep). Thus, a reference axis may be obtained from one or more of the cross product vectors.

However, the cross product has a sign which depends on the direction of rotation between the two vectors. The selective inversion removes this ambiguity so that the resulting cross product vectors are normalized. This for example involves selecting a reference cross product vector and inverting cross product vectors which are negative relative to the reference (i.e. those having an angle larger than 90 degrees to the reference).

In one example, the controller is adapted to determine the reference craniocaudal rotation axis as a unity vector having a direction based on an average of the resulting (i.e. inversion corrected) cross product vectors.

The reference craniocaudal axis derived by this process may be directed in the head-to-toe direction or in the toe-to-head direction. The controller is thus preferably adapted to determine the head-to-toe direction of the reference craniocaudal axis relative to the subj ect.

This may be achieved by:
monitoring accelerations during sitting or standing; or
monitoring accelerations caused by vital signs.

These measures enable the correct direction of the reference craniocaudal axis to be identified. The vital signs for example comprise breathing and/or heart beats.

The controller for example determines the rotation angle about the reference craniocaudal rotation axis relative to a default orientation, and the controller is adapted to determine the subject pose corresponding to the default orientation. The orientation corresponding to the supine posture may then be identified.

This is the determination of a reference posture as mentioned above. The subject pose corresponding to the default orientation of the reference craniocaudal rotation may be determined by:
obtaining a calibration measurement during which the subject is supine; or
obtaining a calibration measurement during which the subject taps the accelerometer; or
analyzing a spread of rotation angles over time.

Thus, there are various ways to identify a supine posture either from movements over time or by implementing a calibration procedure. For some subjects, they will lie generally within a range of 90 degrees either side of supine, whereas some subjects may lie prone (i.e., on their stomach) during the night (in addition to having been in a supine posture). Thus, in some cases automatic approaches may be used requiring no user interaction. In other cases, ambiguity is present so that a calibration approach which may involve user input can be used.

The invention also provides a positional sleep therapy system comprising:
the system for determining a sleep posture of a subject as defined above; and
an alert system for alerting the subject of the need to change their sleep posture in dependence on the determined sleep posture.

The sleep posture determining system thus enables supine and lateral sleep postures to be identified and this information is used in conventional manner as part of a positional sleep therapy system.

The invention also provides a method of determining a sleep posture of a subject comprising:
receiving three-axis accelerometer signals from an accelerometer coupled to the subject;
determining, from a plurality of three-axis acceleration signals over time, a reference craniocaudal rotation axis; and
determining, for an acceleration measurement of interest, a rotation angle about the reference craniocaudal rotation axis.

This method enables a rotation angle to be derived about a craniocaudal (head-to-toe) rotation axis without requiring a particular sensor position with respect to the body of the subject.

The method may comprise determining the reference craniocaudal rotation axis by:
deriving a respective cross product vector for a plurality of pairs of the three-axis acceleration measurements;
inverting selected cross product vectors such that all resulting cross product vectors are directed within a common semi-sphere; and
obtaining the reference craniocaudal rotation axis from the resulting cross product vectors.

The head-to-toe direction of the reference craniocaudal axis relative to the subject may be determined by:
monitoring accelerations during sitting or standing; or
monitoring accelerations caused by vital signs.

The method may comprise determining the rotation angle about the reference craniocaudal rotation axis relative to a default orientation, and determining the subject pose corresponding to the default orientation of the reference craniocaudal rotation axis.

The subject pose corresponding to the default orientation of the reference craniocaudal rotation axis may be determined by:
obtaining a calibration measurement during which the subject is supine; or
obtaining a calibration measurement during which the subject taps the accelerometer; or
analyzing a spread of rotation angles over time.

The invention also provides a computer program comprising computer program code which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 represents clockwise rotation of an accelerometer about a horizontal axis;
Figure 2 shows a craniocaudal axis in bold and three postures;
Figure 3 shows corresponding gravity vectors;
Figure 4 shows graphs of pose angles which can be constructed from the rotation angles;
Figure 5 shows the angles in a polar plot; and
Figure 6 shows a system for determining a sleep posture of a subject.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for determining a sleep posture of a subject, using a three-axis accelerometer coupled to the subject. A plurality of three-axis acceleration signals of the three-axis accelerometer are analyzed over time and from this analysis a reference craniocaudal rotation axis is derived. For an acceleration measurement of interest (i.e. at a particular point in time) a rotation angle can then be determined about the reference craniocaudal rotation axis, thus indicating a sleep posture relative to that reference craniocaudal rotation axis.

The invention thus uses a three axis accelerometer to determine a sleep posture, but without needing specific or accurate placement of the accelerometer.

The first step is determine the rotation axis about which a subject turns during their sleep. This rotation axis is the craniocaudal rotation axis (the axis through the body from the head to the feet).

An accelerometer that is not accelerating measures the projection of gravity onto its axes. The gravity manifests itself as an acceleration away from the earth, because the gravitational force pulls a mass in the accelerometer downward, which, for the accelerometer, is as if it is accelerating in an upward direction.

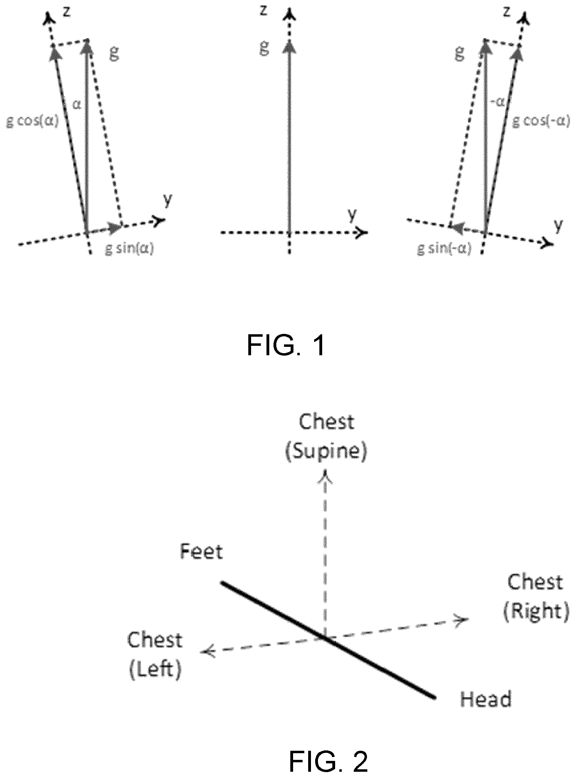

Figure 1 represents clockwise rotation of an accelerometer about a horizontal axis, and hence a rotation of the x, y, z coordinate space of the accelerometer relative to the gravity vector g. The rotation causes the projection of the gravity vector g on the y-axis of the accelerometer coordinate space to change from -g sin(α) to +g sin(α).

During sleep, the various sleeping postures manifest themselves as rotations around the craniocaudal axis.

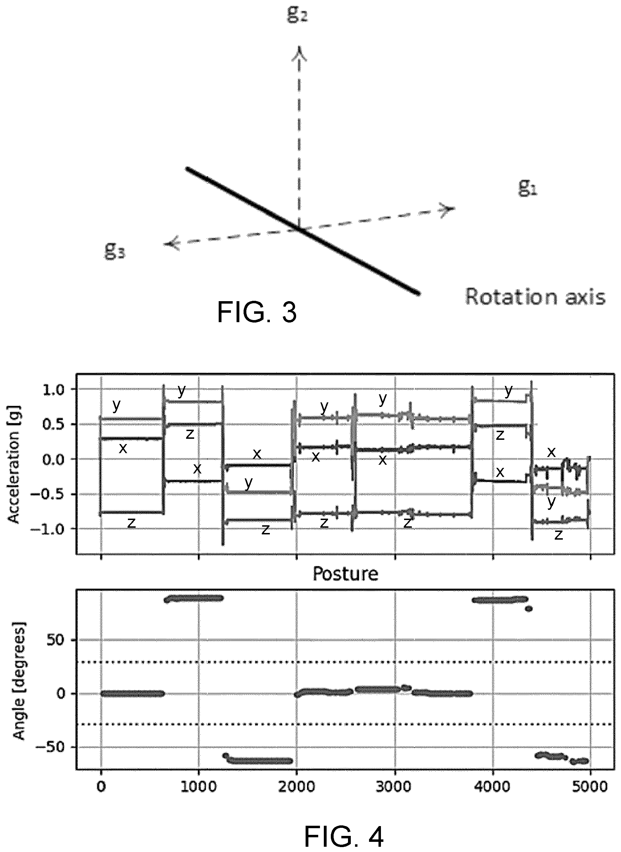

Figure 2 shows the craniocaudal axis in bold and three postures (Left, Supine, and Right) as dashed arrows. Figure 3 shows corresponding gravity vectors g1, g2, g3.

To detect the rotation axis, the projections of the gravity vector on the accelerometer axes, over a time interval, are recorded. These values form a gravity list (g1, g2, ... gn), wherein each item in the list is a 3 dimensional vector of the gravity components projected on the x, y, z axes of the accelerometer coordinate space.

The gravity vectors are all rotations around an axis (which is as yet unknown). Under the assumption that the rotation axis is horizontal, the gravity vectors are perpendicular to it, and the cross product of any two different gravity vectors will align with the rotation axis.

Thus, a reference craniocaudal rotation axis is obtained by deriving a respective cross product vector for a plurality of pairs of the three-axis acceleration measurements.

The cross produce vectors form the elements of an n x n matrix ***M*** of cross products of all pairs of elements (M[i, j] = gi x gj). Each element of ***M*** is thus a three-dimensional vector, of which the magnitude is g sin(angle(gi, gj)).

The direction of this cross product vector depends on the order in which the product is taken (gi x gj = -gj x gi), which is random, as the order between the postures is random.

To remove this randomness, all elements of ***M*** are aligned. For this, a reference element of ***M*** is chosen, and all elements of ***M*** that have an angle to the reference element that is larger than 90 degrees (have a negative dot-product) are inverted. As a result, all elements of ***M*** point into the same half-space (the one defined by the reference element). In this way, selected cross product vectors are inverted such that all resulting cross product vectors are directed within a common semi-sphere.

After this alignment, all vectors in ***M*** are averaged and the result is normalized. This results in a unity vector that represents the rotation axis. Thus, the reference craniocaudal rotation axis is derived from the resulting cross product vectors, as a unity vector having a direction based on an average of the resulting cross product vectors.

The rotation axis corresponds to the craniocaudal axis if the person is lying in bed, independent of rotations of the subject around the axis of gravity. For example, if the person changes from left-diagonal to straight, to right-diagonal (relative to their bed), then this will not affect the posture, as the accelerometer does not measure rotations around the axis of gravity.

In particular, the accelerometer is coupled to the subject, so rotates with the subject when they rotate about the gravity vector, and there is no change to the detected accelerometer signals, based on the assumption that the craniocaudal axis is in the horizontal plane.

To limit computational complexity, and to increase robustness, the elements in the gravity list (g1, g2, ... gn) are for example mean gravities over epochs of time (e.g. units of 30 seconds or 60 seconds), in which the posture is stable.

The craniocaudal axis may for example be determined after an initial period in bed. If insufficient information is available then either a default axis may be used (one from previous usages), or the system may enter an "unknown" state until sufficient information is available. In the "unknown" state no diagnosis or treatment is performed.

Sufficient information is for example available when some turning has taken place, in order to find a rotation axis. The time required is highly dependent on the subject.

The system may for example give a measure of whether sufficient information is available to finish calibration or not (depending on how the subject moves, and/or on past information).

Once the reference axis has been determined, it will remain valid all night as long as the accelerometer remains attached.

The instructions for use of the system may for example prescribe a certain way of attaching the sensor. The system may use this, in combination with data from previous nights, to start with a default axis. The system then learns and adapts over time but it assumes a fixed (or at the most slowly varying) relation between the body posture and the sensor orientation. The sensor design may also mean that there are only a small number of possible ways to wear the sensor, for a given subject. This restricts the degrees of freedom, so that measured signals can be matched to the only possible sensor wearing positions.

After the rotation axis has been identified, the angles of rotation can be determined. For this, a reference gravity vector may be chosen arbitrarily from the gravity list. For each element in the gravity list (representing the gravity vector resolved onto the accelerometer coordinate axes at a particular time period, hence when the accelerometer has a particular orientation), the rotation angle is determined relative to this reference.

The reference and the element (which both are three-dimensional vectors) are projected onto the plane defined by the rotation axis and the angle between the two projections is calculated. This results in an angle for each element in the gravity list (with angle 0° for the reference, as the angle with itself is 0°).

Thus, for an acceleration measurement of interest (i.e. at any particular time of interest) a rotation angle is then obtained about the reference craniocaudal rotation axis and relative to a reference element in the gravity list.

Figure 4 shows graphs which can be constructed from the rotation angles. The x-axis is time, and in this example the subject has 8 different time periods over which the subject orientation is averaged as explained above. Each time period comprises multiple measurement epochs, for example the measurement epochs have a duration of 10 seconds.

The top graph shows the averaged gravity list elements for the time epochs, as separate x, y and z raw acceleration components. The bottom graph shows the calculated rotation angle relative to the reference.

The first gravity vector is taken as the reference, causing the angle to start at 0°.

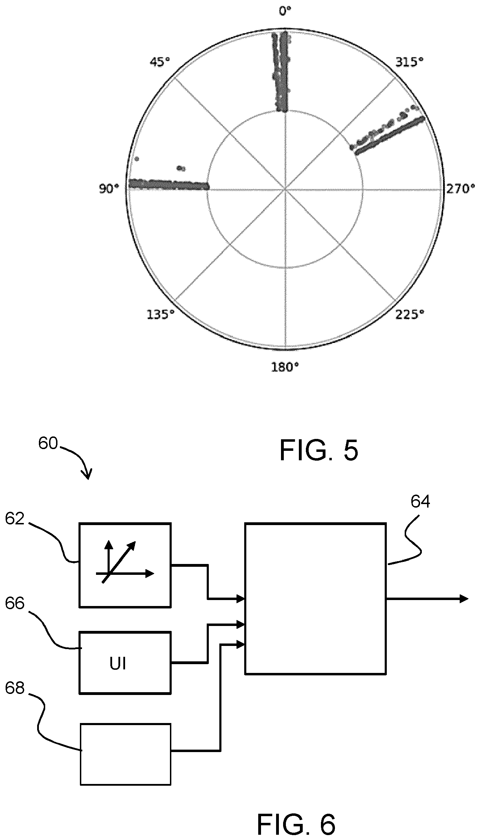

Figure 5 shows the angles in a polar plot. The angle is the rotational angle and the diameter is arbitrary. This graphs gives an indication of the density of postures with particular angle. The polar plot can be considered to be a view along the craniocaudal axis.

The rotational angle is obtained above with respect to a reference posture.

The direction of the craniocaudal axis (i.e. is it in the head-to-toe direction or the toe-to-head direction?) is not yet defined so it is not yet known if a lateral posture is a left side or a right side. The actual posture corresponding to 0° is also not yet determined.

Methods of identifying the direction and reference posture are discussed below. In addition, the system may use information from previous nights as default identifications, as it is likely that sensor position and orientation do not change significantly from night to night.

If totally random sensor placement is possible, it is not possible to rely on prior orientation information. When relying on prior orientation and habitual behavior of the user, a level of uncertainty is present, but this gradually reduces over the night while new data comes in.

The craniocaudal axis direction can be identified in various ways.

By performing detection when the subject is sitting up, or walking, gravity will align with the cranial direction (and not with the caudal direction).

The detection of expected vital signs on an axis concordant with (in the case of a lying posture) or perpendicular to (in the case of sitting/standing) the detected gravity components may also be used. These motions are generally perpendicular to the surface of the chest/abdomen. This can be achieved by detecting the presence of strong periodic components (breathing, heart beats) in the signal, e.g. by means of a time-frequency analysis.

The reference posture, and hence the angle corresponding to the supine posture, can also be identified in various ways.

The subject may for example be asked to lie in a supine posture when the therapy/recording/analysis starts. This can be used as an anchor point to determine the remaining postures.

As accelerometers are sensitive to user interaction such as tapping; this may be used to define the supine posture.

The accelerometer will have a particular response to tapping, and this response enables the orientation of the accelerometer relative to gravity to be identified.

If the supine posture has not been defined then the sensor may trigger the user to perform a tapping. Also, if the reference posture has been set wrongly, then the patient may notice this and may correct it.

Instead of requiring involvement of the subject, there may be automatic calibration. If the automatically detected lying postures span a pre-defined range (e.g. 180 degrees), and if it assumed (or known from user history) that one of the postures is supine, the remaining postures can be determined automatically. For example, it can be assumed that the 0 degree angle in Figure 5 is the supine posture without any further input from the subject.

There may also be a semi-automatic calibration. If the range of postures spans an ambiguous range (e.g. 90 degrees), a simple user interaction method could be used to infer which postures the subject slept on; for example by asking the subject at the end of the morning whether they slept with a specific posture, e.g. on the left side. This would disambiguate the remaining postures.

If the postures span 360 degrees, in which case the user slept both in a prone and in a supine posture, the range will again be ambiguous. The system could automatically determine this and either ask for user input, or revert to a "manual calibration" approach (e.g. tapping, or preset positions at the beginning of the night) in subsequent nights.

As another example if the performance (quality) in the automatic detection of a specific vital sign is lower when in a specific posture (e.g. it may be more difficult to measure heart beats when the subject is lying on the left side), an automatically determined quality indicator (e.g. % of plausible interbeat intervals) could be computed per posture (e.g. average quality for a group of measurements corresponding to one particular detected subject pose), and based on the average quality, simple rules could be used to disambiguate the lying postures.

A calibration may also involve design restriction. If the different accelerometer placement configurations by the user are limited by construction (e.g. the casing is always placed horizontally, parallel to the chest, only potentially turned upside down, or backward/reversed), this additional information could be used to disambiguate the detected postures

Tracking transitions between the postures may be used. This is because the user will reverse the rotation direction between moving to a side posture, and moving away from that side posture.

Figure 6 shows a system 60 for determining a sleep posture of a subject, comprising:
a three-axis accelerometer 62 for coupling to the subject; and
a controller 64.

The controller is adapted to determine, from a plurality of three-axis acceleration signals of the three-axis accelerometer over time, a reference craniocaudal rotation axis. It then determines, for an acceleration measurement of interest (i.e. the acceleration measurement at any particular time of interest) a rotation angle about the reference craniocaudal rotation axis.

Figure 6 also shows a user interface 66 and a vital signs sensor, one or both of which may be used to provide additional input to assist the system in determining the pose corresponding to the reference orientation about the craniocaudal axis, as explained above.

The invention is of particular interest for positional sleep apnea therapy. However, it may be used for any application that may make use of body posture information while in bed. Examples are systems for snoring detection or for monitoring susceptibility to bed sores.

When used within a positional sleep therapy system, the system for determining a sleep posture is combined with an alert system for alerting the subject of the need to change their sleep posture in dependence on the determined sleep posture.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system (60) for determining a sleep posture of a subject, comprising:
a three-axis accelerometer (62) for coupling to the subject; and
a controller (64),
wherein the controller is adapted to:
determine, from a plurality of three-axis acceleration signals of the three-axis accelerometer over time, a reference craniocaudal rotation axis; and
determine for an acceleration measurement of interest a rotation angle about the reference craniocaudal rotation axis

2. The system of claim 1, wherein the controller (64) is adapted to determine the reference craniocaudal rotation axis by:
deriving a respective cross product vector for a plurality of pairs of the three-axis acceleration measurements; and
inverting selected cross product vectors such that all resulting cross product vectors are directed within a common semi-sphere; and
obtaining the reference craniocaudal rotation axis from the resulting cross product vectors.

3. The system of claim 2, wherein the controller (64) is adapted to determine the reference craniocaudal rotation axis as a unity vector having a direction based on an average of the resulting cross product vectors.

4. The system of any one of claims 1 to 3, wherein the controller (64) is adapted to determine the head-to-toe direction of the reference craniocaudal axis relative to the subj ect.

5. The system of claim 4, wherein the controller (64) is adapted to determine the head-to-toe direction of the reference craniocaudal axis relative to the subject by:
monitoring accelerations during sitting or standing; or
monitoring accelerations caused by vital signs.

6. The system of claim 5, wherein vital signs comprising breathing and/or heart beats.

7. The system of any one of claims 1 to 6, wherein the controller (64) is adapted to determine the rotation angle about the reference craniocaudal rotation axis relative to a default orientation, and the controller is adapted to determine the subject pose corresponding to the default orientation of the reference craniocaudal rotation axis.

8. The system of claim 7, wherein the controller (64) is adapted to determine the subject pose corresponding to the default orientation of the reference craniocaudal rotation by:
obtaining a calibration measurement during which the subject is supine; or
obtaining a calibration measurement during which the subject taps the accelerometer; or
analyzing a spread of rotation angles over time.

9. A positional sleep therapy system comprising:
the system for determining a sleep posture of a subject of any one of claim 1 to 8; and
an alert system for alerting the subject of the need to change their sleep posture in dependence on the determined sleep posture.

10. A method of determining a sleep posture of a subject, comprising:
receiving three-axis accelerometer signals from an accelerometer coupled to the subject;
determining, from a plurality of three-axis acceleration signals over time, a reference craniocaudal rotation axis assumed to lie in a horizontal plane; and
determining, for an acceleration measurement of interest, a rotation angle about the reference craniocaudal rotation axis.

11. The method of claim 10, comprising determining the reference craniocaudal rotation axis by:
deriving a respective cross product vector for a plurality of pairs of the three-axis acceleration measurements;
inverting selected cross product vectors such that all resulting cross product vectors are directed within a common semi-sphere; and
obtaining the reference craniocaudal rotation axis from the resulting cross product vectors.

12. The method of claim 10 or 11, comprising determining the head-to-toe direction of the reference craniocaudal axis relative to the subject by:
monitoring accelerations during sitting or standing; or
monitoring accelerations caused by vital signs.

13. The method of any one of claims 10 to 12, comprising determining the rotation angle about the reference craniocaudal rotation axis relative to a default orientation, and determine the subject pose corresponding to the default orientation of the reference craniocaudal rotation axis.

14. The method of claim 13, comprising determining the subject pose corresponding to the default orientation of the reference craniocaudal rotation axis by:
obtaining a calibration measurement during which the subject is supine; or
obtaining a calibration measurement during which the subject taps the accelerometer; or
analyzing a spread of rotation angles over time.

15. A computer program comprising computer program code which is adapted, when said program is run on a computer, to implement he method of any one of claims 10 to 14.
